(19) Europäisches Patentamt
European Patent Office
Office européen des brevets

(11) **EP 4 388 980 A1**

(12) **EUROPEAN PATENT APPLICATION**

(43) Date of publication:
**26.06.2024 Bulletin 2024/26**

(21) Application number: **22215421.3**

(22) Date of filing: **21.12.2022**

(51) International Patent Classification (IPC):
**A61B 5/00** *(2006.01)*      **A61B 5/1455** *(2006.01)*

(52) Cooperative Patent Classification (CPC):
**A61B 5/0013; A61B 5/0022; A61B 5/14552;
A61B 5/7232; G16B 30/20; G16H 80/00;** G06T 9/00

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Koninklijke Philips N.V.
5656 AG Eindhoven (NL)**

(72) Inventors:
• **WANG, Haibo
5656AG Eindhoven (NL)**
• **SPETH, Jeremy
5656AG Eindhoven (NL)**
• **VAN ZON, Cornelis
5656AG Eindhoven (NL)**
• **KIRENKO, Ihor Olehovych
5656AG Eindhoven (NL)**

(74) Representative: **Philips Intellectual Property &
Standards
High Tech Campus 52
5656 AG Eindhoven (NL)**

(54) **COMPRESSION OF VITAL SIGN IMAGE DATA**

(57)      A mechanism for compressing image data encoding vital sign information. The image data undergoes a color space transformation from a first color space to a second color space. In the second color space, data in a first channel set encodes more vital sign information than data in a second channel set. The data in the first channel set is compressed with less loss than the data in the second channel set, to thereby produce compressed image data.

FIG. 1

EP 4 388 980 A1

**Description**

FIELD OF THE INVENTION

The present invention relates to vital sign monitoring, and in particular to vital sign monitoring by means of cameras.

BACKGROUND OF THE INVENTION

In the healthcare domain, there is an ongoing interest in the accurate estimation of vital signs of a patient. Examples of vital signs are well known to the skilled person, and include: temperature, pulse rate, respiration rate, SpOz levels, blood pressure and so on.

There is also a growing interest in telehealth, in which a subject/patient communicates remotely with a clinician using a camera-based system. Telehealth can not only significantly reduce the risk of infectious disease spreading, but also breaks the restrictions posed by physical distance, allowing better sharing of medical resources with subjects/patients in need of care.

One major issue to the widespread uptake of telehealth systems is the absence of medical-grade wearable devices and/or medical assistants available to conduct vital sign measurements. A proposed approach to at least partially overcoming this issue is to make use of a camera, responsive to visible and/or infrared light, to generate digital image data (e.g., a single image or video data) of the subject and determine one or more vital signs from the image data. Such cameras can be labelled "vital sign cameras", which are thereby configured to generate "vital sign image data" that can be appropriately processed to determine one or more vital signs, i.e., vital sign information. As a camera is a natural component of telehealth systems, vital sign cameras can fit seamlessly into such systems.

Vital sign cameras are also advantageous outside of the context of telehealth systems. For example, when treating neonates and subjects with one or more burns, it is preferred to avoid contact-based sensors. Sensing with a camera also allows for remote monitoring (e.g., to reduce a risk for contagious/infectious diseases) and/or can allow a single monitoring device (i.e., a single camera) to monitor a plurality of individuals simultaneously.

There is an ongoing desire to improve the efficiency, speed and reliability of operating a vital sign camera without significantly affecting or reducing the accuracy of vital sign data/information extractable therefrom.

SUMMARY OF THE INVENTION

The invention is defined by the claims.

According to examples in accordance with an aspect of the invention, there is provided a computer-implemented method for compressing vital sign image data, being image data in a first color space from which vital sign information of a subject is derivable, to produce compressed image data for transmittal over a communication channel.

The computer-implemented method comprises: receiving the vital sign image data; transforming the vital sign image data into intermediate image data in a second, different color space, the intermediate image data comprising data in a plurality of channels formed of a first channel set and a second channel set, each channel set comprising one or more channels; and performing a compression procedure to produce the compressed image data.

The compression procedure comprises: performing a first compression process on the data in the first channel set; and performing a second compression process on the data in the second channel set.

The data in the first channel set encodes more vital sign information than the data in the second channel set; and the first compression process is less lossy than the second compression process.

It is recognized that it would be advantageous to process the vital sign image data at a separate processing system (e.g., a cloud-computing system) to that associated with the camera that captures the vital sign image data. However, the direct transmittal of the "raw" vital sign image data to the separate processing system would require significant bandwidth. There is therefore a need/desire to compress the vital sign image data before sending it on to the separate processing system, e.g., via a bandwidth constrained network.

However, it has been identified that existing compression techniques of image data would unacceptably degrade the quality of the vital sign image data to the extent that derivation of vital sign information from compressed (vital sign) image data cannot be accurately performed.

The present disclosure proposes a new compression technique to overcome this issue. In particular, the proposed approach converts the vital sign image data into a different color space, in which one or more channels are more responsive to vital sign information than one or more other channels, i.e., they carry more data relevant to the vital sign than the other channel(s). A more lossy compression technique is performed on those the less relevant channel(s) compared to the more relevant channel(s).

This approach provides a technique that achieves compression of vital sign data without significant loss of relevant vital sign information as a result of the compression procedure.

Embodiments can make use of existing color-space transforms in performing the compression of vital sign image data, in particular any reversible color-space transforms that are used in existing vital sign information extraction procedures or by adapting non-reversible color-space transforms to become reversible (e.g., through addition of one or more additional channels for the transformation).

The present invention also recognizes that a color-space transform is a relatively simple procedure that can be carried out by a processor associated with a camera, allowing the more complex task of vital sign extraction to be performed by a different processor with greater processing power or capabilities.

The image data may comprise data for a single image, a sequence of images or a video (i.e., be video data).

The transformation of the vital sign data into the intermediate image data is reversible, such that image data can be reversibly transformed between the first and second color spaces.

The data in the first channel set preferably encodes or contains more vital sign information than any combination of the same number of channels of the vital sign image data. Thus, in such embodiments, if the first channel set comprises X channels, then the data in the first channel set encodes more vital sign information than data in any combination of X channels of the vital sign image data, where X is an integer value.

Preferably, the first channel set encodes color/chrominance information of the vital sign image data and the second channel set encodes luminance information of the vital sign image data. This approach recognizes that, contrary to the assumption of most conventional compression techniques, the luminance component of vital sign image data is less relevant for accurate derivation of vital sign information. Thus, improved compression can be achieved.

Preferably, the first compression process is lossless. This ensures that the data that provides the largest contribution to vital sign information remains intact as a result of compression, reducing noise introduced into the vital sign information during transmission over the communication channel.

The step of transforming the vital sign image data may comprise processing the vital sign image data using a color space transformation derived from a transformation performed as part of a vital sign extraction procedure performed on vital sign image data. In this way, the performance of a vital sign extraction procedure can effectively be split into a procedure performed as part of a compression approach, and a procedure performed after decompression.

In some examples, the step of transforming the vital sign image data comprises performing a matrix multiplication on the vital sign image data. This provides a simple and reversible mechanism for transforming the vital sign image data to the second color space to produce the intermediate image data.

The first color space may be an RGB color space. Thus, the vital sign image data may effectively be raw or standardized image data.

The method may further comprise, for each channel of the first channel set, identifying values of the intermediate image data in said channel that represent exposed skin of the subject; determining a maximum value and a minimum value of the identified values; and scaling each value in the channel using the maximum value and the minimum value.

This approach recognizes that the skin of the subject contains the most important vital sign information within vital sign image data. By effectively scaling the values of at least the first channel set of the intermediate image data using pixel values of the skin of the subject, a quantization error introduced when producing the intermediate image data can be mitigated and/or attenuated.

In some examples, for each channel of the first channel set, the step of scaling each value in the channel comprises, for each value: subtracting the minimum value from the value to produce a first intermediate value; dividing the first intermediate value by the difference between the maximum value and the minimum value to produce a second intermediate value; and multiplying the second intermediate value by a predetermined scaling factor.

This provides a suitable approach for scaling the values of each channel of the intermediate image data to fall within the scale of the skin of the individual.

The first compression process may be a lossless PNG compression process. The second compression process may be a JPEG compression process. The skilled person will appreciate that any other form of compression could be used and/or performed on the first and second channel sets.

The computer-implemented method may further comprising transmitting the compressed image data, e.g., to a different processing system to that that produces the compressed image data or to a memory/storage system. The transmission may take place over any suitable communication channel, e.g., via the internet.

If scaling of the values in the channel(s) is performed, then the step of transmitting the compressed image data may further comprise transmitting the determined maximum and minimum value for each channel. This facilitates reversal of the scaling procedure by the processing system that receives the compressed image data. The transmittal of the maximum and minimum values does not significantly increase the bandwidth requires for transmittal of the compressed image data, as they represent a small proportion of the total data that is transmitted.

There is also proposed a computer-implemented method for generating vital sign information of a subject. The computer-implemented method comprises: compressing, at a first processing system, vital sign image data by performing any previously described method to produce compressed image data; transmitting the compressed image data to a second, different processing system; performing, at the second processing system, a decompression procedure on the compressed image data to produce reconstructed intermediate image data; and processing the reconstructed intermediate image data to generate vital sign information of the subject.

The second processing system is preferably a cloud-computing system. However, this is not essential. Rather, the second processing system may be located at a same site (i.e., location) as the first processing system or connected to it in a way that does not involve a cloud-based system.

Preferably, the first processing system is a processing system of a camera system that includes the vital sign camera that captured the vital sign image data.

In some examples, processing the reconstructed intermediate image data to generate vital sign information of the subject comprises processing the reconstructed intermediate image data without reconstructing the vital sign image data in the first color space.

There is also proposed a computer program product comprising computer program code which, when executed on a computing device having a processing system, causes the processing system to perform all of the steps of any previously described method. There is also proposed a non-tangible storage medium that stores or comprises the computer program product.

There is also proposed a processing system for compressing vital sign image data, being image data in a second color space from which vital sign information of a subject is derivable, to produce compressed image data.

The processing system is configured to: receive the vital sign image data; transform the vital sign image data into intermediate image data in a second, different color space, the intermediate image data comprising data in a plurality of channels formed of a first channel set and a second channel set, each channel set comprising one or more channels; and perform a compression procedure to produce the compressed image data

The compression procedure comprises: performing a first compression process on the data in the first channel set; and performing a second compression process on the data in the second channel set.

The data in the first channel set is more responsive to changes in the vital sign information than the data in the second channel set; and the first compression process is less lossy than the second decompression process.

There is also proposed a distributed processing system for generating vital sign information of a subject, the distributed processing system comprising a first processing and a second, different processing system.

The first processing system is the processing system previously described and further configured to transmit the compressed image data to the second processing system.

The second processing system is separate to the first processing system and is configured to perform a decompression procedure on the compressed image data to produce reconstructed intermediate image data; and process the reconstructed intermediate image data to generate the vital sign information of the subject.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 is a flowchart illustrating a method;
Fig. 2 illustrates a relationship between a first color space and a second color space;
Fig. 3 is a flowchart illustrating another method; and
Fig. 4 illustrates a distributed processing system.

DETAILED DESCRIPTION OF EXEMPLARRY EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a mechanism for compressing image data encoding vital sign information. The image data undergoes a color space transformation from a first color space to a second color space. In the second color space, data in a first channel set encodes more vital sign information than data in a second channel set. The data in the first channel set is compressed with less loss than the data in the second channel set, to thereby produce compressed image data.

One disadvantage of determining vital sign information from vital sign image data (such as video data) is that a significant amount of storage and processing power is required to accurately analyze the vital sign image data to produce the vital sign information. This places a significant burden/strain on the camera capturing system, which typically has a relatively low powered processing system and/or relatively small memory.

This problem is usually overcome by transmitting the vital sign image data from the camera system to an external processing system, such as a cloud-computing system or the like, over a communication channel. It is usually considered necessary to compress the vital sign image data for transmission over the communication channel, to facilitate "real-time" analysis and generation of vital sign information.

However, existing compression techniques may introduce significant coding artefacts to an image, which can severely damage the underlying vital sign information encoded in the vital sign image data.

An existing procedure for producing vital sign information includes at least the following steps: image capture; image compression; transmission; image decompression; and vital sign information production.

Some procedures may also include a step of vital sign information preservation between image capture and image compression and/or a step of vital sign information restoration between image decompression and vital sign information production. These additional steps typically aim to overcome the problems introduced by image compression.

Image capture includes the capturing and recording of image data (e.g., video data) of a subject. The image data is vital sign image data, in that it contains information that can be processed to identify vital sign information of the subject. Whilst it is important to ensure low noise capture of the vital sign image data, by careful selection of camera settings, modifications or improvements at this step would not directly solve the problems introduced by compression.

If performed, a step of vital sign information preservation includes locating one or more locations in the image data that respond to changes in vital sign information and amplifying the corresponding portions of the image data to give a higher amplitude response during later vital sign information production from vital sign derivation algorithms. This step can mitigate negative compression effects by allowing the enhanced vital sign information to pass through, even if the video is still slightly damaged. One of the difficulties with performing this modification, is that the amplified image data would be transformed in different manners depending on the compression algorithm. This may require a different preservation scheme for each compression codec.

The image decompression process/step is entirely dependent on the chosen encoding scheme and is typically designed to reconstruct perceptually similar image data to the original vital sign image data. Thus, image decompression receives compressed (and transmitted) image data and produces reconstructed image data (being a reconstruction of the original vital sign image data).

If performed, vital sign information restoration aims to transform the reconstructed image data to at least partially restore the original vital sign information contained in the original vital sign image data (which may have been attenuated during compression). This step is similar to the step of vital sign information preservation, but the modified image data will be processed to generate vital sign information (rather than being compressed). It has been suggested that performing a super-resolution on reconstructed image data can improve the accuracy of subsequent vital sign information production.

Vital sign information production computes or determines vital sign information from the reconstructed image data (which has optionally been processed by a vital sign information restoration process). Thus, this process effectively extracts vital sign information from the reconstructed image data. It has been suggested that improvements to this step can be achieved by using data-driven deep learning approaches to process the reconstructed image data and produce the vital sign information.

From the foregoing, it will be clear that improvements to steps of the procedure for producing vital sign information can at least partially overcome the problems introduced by image compression.

The present disclosure proposes a new technique for generating compressed image data by processing the vital sign image data, including the step of image compression. This will have a natural impact, in the processing pipeline, on a corresponding process of reconstructing the vital sign image data from the compressed image data, including at least the step of image decompression.

The present disclosure appreciates that although existing and developed video compression algorithms have substantially improved in terms of perceptual quality for the human visual system, they are detrimental to remote vital sign estimation. In fact, it has been recognized that perceptual quality and remote vital sign estimation are nearly orthogonal/opposite in their aims. For instance, the human visual system is more sensitive to luminance than chrominance, so existing compression algorithms typically compress chrominance more than luminance. The present disclosure recognizes that vital sign information in image data is typically manifested in the form of chrominance, and that luminance changes can (in practice) be a source of noise.

The present invention proposes a new compression scheme that is designed for preserving vital sign information when compressing vital sign image data for transmittal over a communication channel.

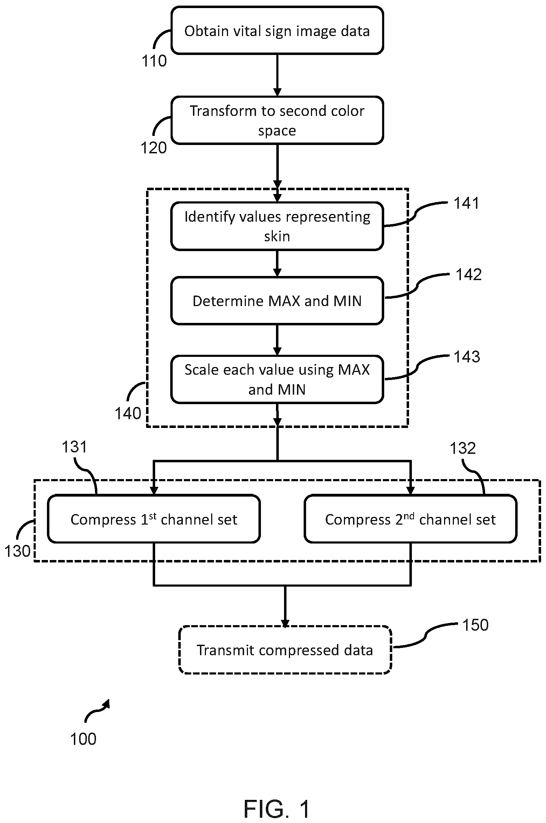

Fig. 1 is a flowchart illustrating a computer-implemented method 100 according to an embodiment. The computer-implemented method may be performed by a processing system.

The method 100 comprises a step 110 of receiving vital sign image data. The vital sign image data is image data in a first color space from which vital sign information of a subject is derivable. For instance, the vital sign image data may comprise video data of a face of a subject which can be processed to identify or determine a pulse rate of the subject. As another example, the vital sign image data may contain video data of a skin of a subject that can be processed to identify or determine an SpOz value of the subject. Other suitable forms of vital sign image data would be apparent to the skilled person.

The vital sign image data is produced by a camera system that records image data of the subject. Examples of suitable camera systems are well known in the art.

The first color space may be any appropriate color space in which an image is captured. For instance, the first color space may be an RGB color space.

The method 100 also comprises a step 120 of transforming the vital sign image data into a second, different color space. This produces intermediate image data. Thus, step 120 performs a color space transformation on the vital sign image data to produce intermediate image data. The intermediate image data comprises data in a first channel set and a second channel set, each formed of one or more channels. Each channel set contains different one or more channels.

The data in the first channel set encodes more vital sign information than the data in the second channel set. Examples of suitable color space transformation procedures to achieve this are provided later in the disclosure. As an example, the first channel set may encode color/chrominance information and the second channel set may encode luminaire information, as it has been recognized that vital sign information is typically manifested in the form of color/chrominance (rather than luminance).

Put another way, the data in the first channel set is more responsive to vital sign information than data in the second channel set. Thus, all other variations put aside, if the subject in first vital sign image data has different vital sign information than in second vital sign image data, then the difference in the data in the first channel set (between that derived from the first vital sign image data to the second vital sign image data) will be larger than the difference in the data in the second channel set (between that derived from the first vital sign image data to the second vital sign image data).

Preferably, the vital sign information is derivable from the data carried by the first channel set alone. Thus, the color space transformation may be configured to encode the vital sign information primarily in the first channel set, such that the first channel set carries sufficient data to permit derivation of (accurate) vital sign information.

Similarly, preferably the data carried or encoded by the second channel set is redundant for deriving the vital sign information. In other words, the data in the second channel set could be subject to noise or other coding artefacts without significantly affecting the capability of deriving the vital sign information for the subject.

It will be clear that the data in the first channel set encodes or contains more vital sign information than any combination of the same number of channels of the vital sign image data. Thus, if the first channel set comprises X channels, then the data in the first channel set encodes more vital sign information than data in any combination of X channels of the vital sign image data. X is an integer value.

Put another way, the color space transformation concentrates most or all of the vital sign information encoded in the vital sign image data into a subset (i.e., not all) channels of a second color space.

The color space transformation performed in step 120 is reversible. Thus, it is possible to perform lossless, or near lossless, transformation of the vital sign image data between the first and second color spaces. Assuming that a linear transformation procedure is used, this can be achieved by ensuring the transformation matrix for performing the linear transformation is square (e.g., 3x3) and full rank. Other restrictions/adaptations for other forms of transformation will be apparent to the skilled person.

If the vital sign image data is in the form of video data, i.e., a sequence of frames of image data, then each frame may be individually transformed into a/the second color space. Thus, each frame of vital sign video data may represent an instance of vital sign image data to be color space transformed in step 120.

The method 100 also comprises a step 130 of performing a compression procedure on the intermediate image data. This produces compressed image data for transmittal over the communication channel.

The compression procedure comprises performing, in a sub-step 131, a first compression process on the data in the first channel set; and performing, in a sub-step 132, a second compression process on the data in the second channel set. The first compression process 131 is less lossy than the second compression process.

Thus, at least some data is lost by the second compression process. However, as the data in the first channel set encodes more vital sign information than the data in the second channel set, the compressed image data can be subsequently constructed (i.e., decompressed) whilst retaining significant vital sign information about the subject for later processing.

It has previously been described how a color space transformation is performed on the vital sign image data to produce intermediate image data.

The color space transformation is preferably such that the first channel set encodes color/chrominance information of the vital sign image data and the second channel set encodes luminance information of the vital sign image data. However, this is not essential.

Various examples of suitable color space transformations are hereafter described.

In general, a color space transformation can be defined by a transformation matrix and an optional biasing vector. The transformation matrix is used to multiply a first color space pixel, such as an RGB pixel [R, G, B], to produce a second color space pixel $[C_1, C_2, C_3]$. This can be performed for each pixel of the vital sign image data to produce the intermediate image data. Of course, the biasing vector may define biasing values for each value in the second color space pixel (e.g., a biasing value to add/subtract for each value of any pixel in the second color space).

The transformation matrix and/or the biasing vector may be used/defined to keep or maintain the values of pixels in the second color space (i.e., the values of pixels of the intermediate image data) within a particular range (e.g., of from 0 to 255). This range may, for instance, be a maximum allowable range of values for pixels of the vital sign image data.

This range may be seen as a requirement for accurate compression of the intermediate data, as compression techniques may assume or be designed to operate under the conditions (e.g., pixel sizes) that would be expected for conventional image data.

For instance, image pixels are typically stored as tuples of unsigned 8-bit integers, which support values in [0,255]. Transforming image data in the first color space (e.g., RGB image data) into the intermediate data in the second image space should therefore be controlled to ensure that the image pixels in the intermediate image data are stored as tuples in the range [0,255].

Of course, different types of image data and different compression strategies have different configurations (e.g., different sizes of allowable ranges) requirements, and this restriction on allowable values for the pixels is not essential.

Put mathematically, the color space transformation, for each pixel of vital sign image data, can be defined as follows:

$$\begin{bmatrix} C_1 \\ C_2 \\ C_3 \end{bmatrix} = A \begin{bmatrix} R \\ G \\ B \end{bmatrix} + D \qquad (1)$$

where A is a 3x3 matrix and D is a 3x1 vector (of the same size as a pixel in the first color space).

The following examples clearly demonstrate how a color space transformation can be constructed from transformations or calculations used in a wide variety of vital sign information determination procedures. Thus, the general principle of the proposed method is described and supported by way of a non-exhaustive set of examples. The skilled person would be similarly capable of producing other suitable color space transformations that meet the requirements, e.g., by adapting other forms of vital sign information determination procedures.

A first color space transformation makes use of the CHROM algorithm set out by G. de Haan and V. Jeanne, "Robust Pulse Rate from Chrominance-Based rPPG," in IEEE Transactions on Biomedical Engineering, vol. 60, no. 10, pp. 2878-2886, Oct. 2013, doi: 10.1109/TBME.2013.2266196.

The CHROM algorithm uses knowledge that changes in reflected light as a result of blood volume changes mainly occur in the chrominance of an image, rather than the luminance or intensity. Given a temporally normalized RGB trace (RGB), the pulse signal for a time segment is calculated with:

$$\begin{bmatrix} P_1 \\ P_2 \end{bmatrix} = \begin{bmatrix} 3 & -2 & 0 \\ 1.5 & 1 & -1.5 \end{bmatrix} RGB \qquad (2)$$

$$S = P_1 - \frac{\sigma(P_1)}{\sigma(P_2)} \cdot P_2 \qquad (3)$$

To use the CHROM algorithm to define a color space transformation, it is first noted that the transformation is not full rank, since it only makes use of a 2×3 matrix. To create a proper and reversible color space transformation, it is possible to determine an orthogonal vector by taking the cross product of the two vectors, $V_3 = V_1 \times V_2 = [3, 4.5, 6]$, where $V_1 = [3, -2, 0]$ and $V_2 = [1.5, 1, -1.5]$.

Given these row vectors, the normalized transformation matrix can be calculated and used to process a value [R, G, B] in an 3-channel RGB color space (e.g., a first color space) into a value $[C_1, C_2, C_3]$ in a 3-channel CHROM space:

$$\begin{bmatrix} C_1 \\ C_2 \\ C_3 \end{bmatrix} = \begin{bmatrix} 0.6 & -0.4 & 0 \\ 0.242 & 0.364 & -0.394 \\ 0.222 & 0.333 & 0.444 \end{bmatrix} \begin{bmatrix} R \\ G \\ B \end{bmatrix} + \begin{bmatrix} 102.4 \\ 100.848 \\ 0 \end{bmatrix} \qquad (4)$$

Since the transformation matrix above is orthonormal, it is also invertible, which allows transformation back to the RGB space, producing a value [R', G', B']:

$$\begin{bmatrix} R' \\ G' \\ B' \end{bmatrix} = \begin{bmatrix} 1.154 & 0.7 & 0.621 \\ -0.769 & 1.050 & 0.931 \\ 0 & -1.138 & 1.241 \end{bmatrix} \begin{bmatrix} C_1 - 102.4 \\ C_2 - 100.848 \\ C_3 \end{bmatrix} \qquad (5)$$

For the CHROM algorithm, after transformation using equation (4), only the first and second channels are pertinent or relevant for vital sign information estimation. Thus, considering the CHROM algorithm as a baseline estimator, the third channel ($C_3$) contains the least pulse information, and can be more compressed heavily without loss of vital sign information.

A second color space transformation makes use of the Plane-Orthogonal-to-Skin (POS) algorithm set out by Wang, W., den Brinker, A. C., Stuijk, S. & de Haan, G. Algorithmic principles of remote ppg. IEEE Trans. Biomed. Eng. 64, 1479-1491 (2016).

The POS algorithm defines a projection plane in RGB space with only soft knowledge requirements, since variance in the skin color does not change the projected values. Given a temporally normalized RGB trace (RGB), the pulse signal for a time segment is calculated with:

$$\begin{bmatrix} P_1 \\ P_2 \end{bmatrix} = \begin{bmatrix} 0 & 1 & -1 \\ -2 & 1 & 1 \end{bmatrix} RGB \qquad (6)$$

$$S = P_1 - \frac{\sigma(P_1)}{\sigma(P_2)} \cdot P_2 \qquad (7)$$

To use the POS algorithm to define a color space, it is first noted that the transformation is not full rank, since it only makes use of a 2×3 matrix. This is similar to the recognition for the CHROM algorithm. To create a proper and reversible color space transformation, it is possible to determine an orthogonal vector by taking the cross product of the two vectors, $V_3 = V_1 \times V_2 = [2, 2, 2]$, where $V_1 = [0, 1, -1]$ and $V_2 = [-2, 1, 1]$. Given these row vectors, we define the normalized transformation matrix into a 3-channel POS space:

$$\begin{bmatrix} C_1 \\ C_2 \\ C_3 \end{bmatrix} = \begin{bmatrix} 0 & 0.5 & -0.5 \\ -0.5 & 0.25 & 0.25 \\ 0.333 & 0.333 & 0.333 \end{bmatrix} \begin{bmatrix} R \\ G \\ B \end{bmatrix} + \begin{bmatrix} 128 \\ 128 \\ 0 \end{bmatrix} \qquad (8)$$

Since the transformation matrix above is orthonormal, it is also invertible, which allows transformation back to RGB space:

$$\begin{bmatrix} R' \\ G' \\ B' \end{bmatrix} = \begin{bmatrix} 0 & -1.333 & 1 \\ 1 & 0.667 & 1 \\ -1 & 0.667 & 1 \end{bmatrix} \begin{bmatrix} C_1 - 128 \\ C_2 - 128 \\ C_3 \end{bmatrix} \qquad (9)$$

In a similar manner to the CHROM approach, after transformation using equation (8), only the first and second channels are pertinent to vital sign information estimation. Considering the POS algorithm as a baseline estimator, the third channel contains the least vital sign information, and can be more compressed heavily without loss of vital sign information.

A third color space transformation makes use of the pulse blood volume (PBV) algorithm set out in G. de Haan and A. van Leest, "Improved motion robustness of remote- PPG by using the blood volume pulse signature," Physiol. Meas., vol. 35, no. 9, pp. 1913-1922, Oct. 2014.

The PBV algorithm was developed using the spectral response of the blood volume PPG signal in RGB space.

After integrating the spectral response over the wavelength, a single linear transformation, the PBV signature, is defined as the vector $V_1$ = [0.33, 0.78, 0.53]. The pulse signal S can then be calculated as:

$$S = kV_1(C_n C_n^T)^{-1} \tag{10}$$

where k is a normalizing constant and $C_n$ is the temporally normalized RGB trace (i.e., the spatial average of pixel values over time).

Although the PBV transformation is useful for estimating the 1-D pulse signal, it requires some modification to be usable for defining a color space transformation for producing an appropriate color space. For the purposes of defining a transformation matrix for a color space transformation, one goal is to find two orthogonal vectors. Since there is no other vital sign specific information from the PBV algorithm, it is possible to arbitrarily select two orthogonal vectors on the plane orthogonal to $V_1$. Thus, first define a random vector $V_2$, then perform the following with Gram-Schmidt:

$$V_2 = V_2 - \frac{(V_2 \cdot V_1)V_1}{\|V_1\|} \tag{11}$$

$$V_3 = V_1 \times V_2 \tag{12}$$

Given these two additional vectors a color transformation matrix $A = [V_1^T \; V_2^T \; V_3^T]$ and a biasing term/terms can be defined or identified (optionally: to keep resulting values in [0,255]. This transforms the vital sign image data to a new 3-channel PBV color space:

$$\begin{bmatrix} C_1 \\ C_2 \\ C_3 \end{bmatrix} = \begin{bmatrix} 0.201 & 0.476 & 0.323 \\ 0.527 & -0.324 & 0.149 \\ 0.278 & 0.222 & -0.5 \end{bmatrix} \begin{bmatrix} R \\ G \\ B \end{bmatrix} + \begin{bmatrix} 0 \\ 23.9 \\ 128 \end{bmatrix} \tag{13}$$

For this transformation, channels $C_2$ and $C_3$ contain less vital sign information than channel $C_1$, and can therefore be compressed to a greater extent without loss of significant vital sign information.

It will be appreciated that the numeric values identified in equation (13) depend upon the random vector $V_2$ that has been selected, and that other random vectors will produce different matrices.

It will also be appreciated that the values in the transformation matrix have been scaled, and the biasing term(s) selected, to ensure that the output values of in the PBV color space meet designed requirements (e.g., fall within a range of from 0 to 255). Thus, it is noted that the channel $C_1$ corresponds to a normalized version of the PBV signature $V_1$.

Since the transformation matrix is orthonormal, it is also invertible, which allows transformation from the PBC color space back to the RGB color space:

$$\begin{bmatrix} R' \\ G' \\ B' \end{bmatrix} = \begin{bmatrix} 0.542 & 1.301 & 0.738 \\ 1.282 & -0.8 & 0.590 \\ 0.871 & 0.367 & -1.328 \end{bmatrix} \begin{bmatrix} C_1 - 0 \\ C_2 - 23.9 \\ C_3 - 128 \end{bmatrix} \tag{14}$$

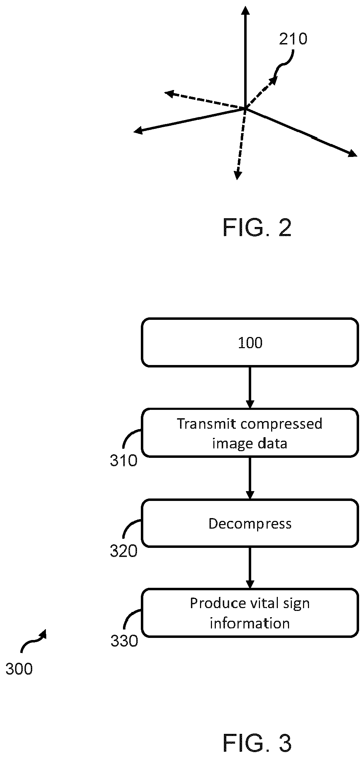

Fig. 2 conceptually illustrates the relationship between a first color space in the form of an RGB color space and a second color space in the form of a PBV color space.

Solid lines represent channels in the RGB color space, with dotted lines representing channels in the PBV color space.

Fig. 2 demonstrates how the PBV color space is a simple transformation of the RGB color space. It also demonstrates how certain colors in the RGB color space contribute more to the vital sign information (which is largely contained in the first channel 210 in the PBV color space), which is reflected in values selected for equation (13).

The preceding examples of possible color space transformation are non-exhaustive. More generally, all preceding examples of color space transformation have been derived from vital sign derivation techniques.

It has been previously explained how an appropriate color space transformation is used to create intermediate image data in the second color space. The intermediate data comprises first and second channel sets, for which data in the second channel set contains or encodes less vital sign information than data in the first channel set.

It is proposed to compress data in the second channel set to a greater extent than data in the first channel set. For instance, the data in the first channel set may be compressed losslessly, whereas data in the second channel set may be compressed in a lossy manner.

Compressing a channel set of an image can be performed in a variety of ways, and may depend upon the requirements of the communication channel. Some common compression approaches include Fourier decomposition, discrete cosine decomposition, wavelet decomposition and/or subsampling. In general, these approaches typically discard high frequency spatial information, since the low frequencies tend to be stronger.

Different types of compression techniques offer different amounts of loss when subsequently decompressed.

For instance, different classes of compression processes or strategies facilitate different amounts of loss in compression. For instance, a JPEG compression technique introduces loss of information, whereas PNG compression techniques support lossless compression.

In a working example that makes use of this recognition, the data in the first channel set is compressed using (lossless) PNG compression and the data in the second channel set is compressed using (lossy) JPEG compression.

As another example, compression can be performed by performing sub-sampling or downsampling (with decompression being performed using an upsampling technique). A higher degree of downsampling effectively provides a higher degree of compression, and vice versa.

In a working example that makes use of this recognition, the data in the first channel set is downsampled by a first amount with the data in the second channel set being downsampled by a second, greater amount.

Existing compression techniques often compress one channel set more than another channel set. However, such compression techniques do not take into account which channels sets contain vital sign information, rather they are designed for greater compression of less perceptible (to human eyes) information. The present disclosure can make use of such existing compression techniques to be used to instead compress channels containing more vital sign information less than channels containing less vital sign information. This means that the compressed data can adhere to an expected format for transmission, thereby adhering to transmission standards and not requiring any modification for transmission.

Thus, in some examples, known compression techniques can be used to perform the compression.

If the intermediate image data is in the form of video data, i.e., a sequence of frames of image data, then each frame may be individually compressed by the compression procedure. Thus, each frame of image data may represent an instance of intermediate image data to be compressed in step 130.

Of course, inter-frame or temporal compression could also/otherwise be used.

Preferably, if inter-frame or temporal compression is performed, inter-frame compression is not performed on data in the first channel set but is performed on data in the second channel set.

Thus, in one example, compressing data in the second channel set comprising compressing using an inter-frame or temporal compression procedure, which is not used for compressing data in the first channel set.

Approaches for decompressing data are well-established in the art, and typically include reversing the compression technique.

Turning back to Fig. 1, it has previously been explained how a color space transformation can be controlled such that the (potential) values of pixels in the intermediate data lie in a same range (e.g., [0,M]) as the (potential) values of the vital sign image data.

The color space transformation can lead to quantization error, since the values for each pixel following the color space transformation will, in practice, be assigned to their closest integer, leading to a maximum residual of 0.5. While seemingly small, this can add artificial noise to the intermediate image data and resultant decompression and later analysis thereof.

To reduce the impact of quantisation noise, it is further proposed to perform value scaling (e.g., channel-wise value scaling) based on the statistical properties of the pixel values for the skin of the subject in the second color space. It is recognized that vital sign information is carried in only those pixels that represent the skin of a subject, as it is the properties of the subject's skin that change responsive to changes in vital signs (e.g., rather than their clothing or the like).

This can be performed in an optional scaling process 140, which is performed for each channel in the at least the first channel set of the intermediate image data. The scaling process 140 may also be performed for each channel of the second channel set, but this is not essential and can be omitted for processing efficiency (as the second channel set does not carry substantial vital sign information). The scaling process may form part of the compression procedure to produce the intermediate image data.

The scaling process comprises a step 141 of identifying values (in said channel) of the intermediate image data that represent exposed skin of the subject; a step 142 of determining a maximum value MAX and a minimum value MIN of the identified values; and a step 143 of scaling each value in the channel using the maximum value and the minimum value.

Approaches for performing step 141 will be apparent to the skilled person.

In particular, step 141 may comprise processing the intermediate image data (or the vital sign image data) to identify one or more regions containing skin of the subject, e.g., containing a face of the subject. As the color space transformation does not change the position of objects within an image, identifying any such regions in the vital sign image data is functionally equivalent to performing the same using the intermediate image data. The values of the identified region(s) can be trivially identified/extracted.

Suitable objection detection techniques for detecting regions of skin in image data are well established in the art, e.g., as suggested by Chaves-González, Jose M., et al. "Detecting skin in face recognition systems: A color spaces study." Digital signal processing 20.3 (2010): 806-823 or Zhang, Shifeng, et al. "Detecting face with densely connected face proposal network." Neurocomputing 284 (2018): 119-127.

Step 142 is performed trivially by using standard maximum/minimum identification techniques using the identified values.

Step 143 may comprise subtracting the minimum value from the value to produce a first intermediate value; dividing the first intermediate value by the difference between the maximum value and the minimum value to produce a second intermediate value; and multiplying the second intermediate value by a predetermined scaling factor.

Mathematically, this can be represented by the following equation:

$$X'_{i,j} = M\left(\frac{X_{i,j} - \min(X)}{\max(X) - \min(X)}\right) \tag{15}$$

where $X_{i,j}$ is the original pixel value, $min(X)$ and $\max(X)$ are the minimum and maximum values of the skin for that channel, and $X'_{i,j}$ is the shifted and scaled version of the image channel. I,j represent all pixels in the intermediate image data. M is the maximum allowable value for pixel values of image data for compression.

It is noted that the skin pixels will only occupy a subspace of the full second color space. The transformation using equation (15) thereby spreads the pixel values of the intermediate data across the permissible [0, M] sampling grid. When rounding to the nearest integer, quantization has a much smaller effect in the scaled space.

For the sake of completeness, during decompression, the values can be trivially transformed back to the original scale with:

$$X_{i,j} = (\max(X) - \min(X))\left(\frac{X'_{i,j}}{255}\right) + \min(X) \tag{16}$$

The only additional bandwidth requirement during transmission of the compressed image data is the sending of the minimum and maximum channel values for each instance of compressed image data, which is negligible.

Although not illustrated, method 100 may further comprise performing vital sign information preservation (e.g., on the obtained vital sign image data between steps 110 and 120).

Method 100 may further comprise a step 150 of transmitting the compressed image data, i.e., the data output by the compression process 130.

If method 100 is performed by a first processing system, then step 150 may comprise transmitting the compressed image data over a communication channel to a second, different processing system.

The second processing system may, for instance, be a cloud-processing system, a server (e.g., at the same or different site as the first processing system) and/or any other processing system.

It is not essential the step of transmitting the compressed image data from the first processing system to the second processing system occur in a single process. Rather, the transmitting may comprise storing the compressed image data in a database or other memory and later accessing/retrieval of the stored compressed image data by the second processing system.

The transmittal of the compressed image data takes place over a communication channel. The communication channel may be any wired and/or wireless connection that facilitates communication between two separate processing systems that makes use of any known wired or wireless communication protocol.

Suitable wireless communication protocols that may be used to establish the communication channel include the Internet protocol suite, an infrared link, ZigBee®, Bluetooth®, a wireless local area network protocol such as in accordance with any of the IEEE 802 standards (e.g., the IEEE 802.11 standard), a 3G, 4G or 5G telecommunication protocol, and so on. Other formats will be readily apparent to the person skilled in the art.

If method 100 comprises performing a scaling process 140, then step 150 may further comprise transmitting the minimum and maximum channel values for each instance of compressed image data.

Fig. 3 is a flowchart illustrating a method 300 for generating vital sign information of a subject that makes use of a previously described method for compressing vital sign image data.

The hardware and/or software-implemented method 300 comprises a process 100 of compressing, at a first processing system, vital sign image data. This is performed using a previously described method of compressing vital sign image data to produce compressed image data. The first processing system may be a processing system of a camera system that originally obtained or captured the vital sign images data.

The method 300 also comprises a step 310 of transmitting the compressed image data to a second, different processing system. Approaches for transmitting compressed image data from a first transmission system to a second, different transmission system have been previously described with reference to Fig. 1.

The method 300 also comprises a step 320 of performing, at the second processing system, a decompression procedure on the compressed image data to produce reconstructed intermediate image data.

The decompression procedure performed in step 320 effectively reverses the compression performed by the compression procedure of process 100.

If process 100 includes a scaling procedure (as previously described), the decompression procedure performed in step 320 can similarly include a descaling procedure, e.g., making use of transmitted maximum and minimum values to reverse the scaling procedure.

The method 300 then performs a step 330 of processing the reconstructed intermediate image data to generate vital sign information of the subject, i.e., to extract vital sign information from the reconstructed intermediate image data.

By way of example, step 330 may comprise transforming the reconstructed intermediate image data from the second color space to the first color space, effectively reconstructing the vital sign image data. The reconstructed vital sign image data can then be processed using a conventional vital sign information production technique.

Step 330 may employ any suitable approach for generating vital sign information by the processing of image data.

A wide variety of approaches for performing step 330 are known in the art, such as those disclosed in Chaichulee, Sitthichok, et al. "Cardio-respiratory signal extraction from video camera data for continuous non-contact vital sign monitoring using deep learning." Physiological measurement 40.11 (2019): 115001.

Other documents that suggest or disclose approaches for processing image data to produce vital sign information include:

G. de Haan and V. Jeanne, "Robust Pulse Rate from Chrominance-Based rPPG," in IEEE Transactions on Biomedical Engineering, vol. 60, no. 10, pp. 2878-2886, Oct. 2013, doi: 10.1109/TBME.2013.2266196; Wang, W., den Brinker, A. C., Stuijk, S. & de Haan, G. Algorithmic principles of remote ppg. IEEE Trans. Biomed. Eng. 64, 1479-1491 (2016); and/or G. de Haan and A. van Leest, "Improved motion robustness of remote- PPG by using the blood volume pulse signature," Physiol. Meas., vol. 35, no. 9, pp. 1913-1922, Oct. 2014.

Alternatively, rather than transforming the reconstructed intermediate image data to the first color space, the intermediate image data can be directly processed. This is because the intermediate image data will contain the vital sign information in an extractable form.

In particular, if the transformation to the second color space is produced from an existing vital sign extraction procedure (e.g., the CHROM, POS or PBV algorithms), then the reconstructed intermediate data may already contain data that has been partially transformed for the purposes of such vital sign extraction procedures. This data can then be further processed using the remainder of the vital sign extraction procedure to generate the vital sign information.

Of course, the generated vital sign information can be used for any conventional purpose, such as for display at a user interface, storage in a storage system (e.g., in a medical record associated with the subject), for generation of one or more alerts for the subject (e.g., by comparing the vital sign information to one or more thresholds). Other suitable approaches are well established in the art, and are not essential to achieving the underlying concept of the present disclosure.

Generally, the previously described compression process has assumed that, if the vital sign image data is video data, each frame of the video data is transformed into a same color space, e.g., the same color space transformation is applied to every frame.

A potential improvement to the proposed algorithm is to allow for updates to the color space transformation (i.e., effectively modifying the second color space) based on feedback from a vital sign determination step (e.g., performed by the second processing system) estimation algorithm.

By way of example, the second processing system may perform multiple different vital sign extraction techniques on the decompressed and inverse transformed image data to generate multiple instances of vital sign information. The most accurate or "best" vital sign information can be identified, e.g., based on periodic and/or statistical properties of the extracted vital signal information. The corresponding vital sign extraction technique can be identified to the first processing system. The first processing system may then use the identified vital sign extract technique to modify the color space transformation, i.e., modify the second color space, using a previously described approach.

By doing this, the color space would be effectively rotated or transformed to increase the amount of vital sign information contained with the first channel set, and reducing the amount contained in the channel, improving the performance of the overall methodology.

Thus, the method may comprise, at the second processing system, processing the reconstructed intermediate image data using a plurality of different vital sign extraction techniques to generate a plurality of instances of vital sign information; selecting, at the second processing system, an instance of the vital sign information responsive to one or more statistical and/or periodic properties of the vital sign information; and, at the second processing system, modifying the second color space responsive to the vital sign extraction technique used to produce the selected vital sign information.

Wang, W., den Brinker, A. C. & de Haan, "Single-Element Remote-PPG," in IEEE Transactions on Biomedical Engineering, vol. 66, no. 7, pp. 2032-2043, Jul. 2019, doi: 10. 1 109/TBME.2018.2882396 sets out a single element (SE) algorithm that estimates a pulse of an individual. The SE algorithm performs multiple projections within a region of the color space for image data that is expected to contain pulse information, and selects the best projection based on the periodic and statistical properties of the resultant signal. The identified projection can be used to update the transformation of the vital signal image data into the intermediate image data, to ensure that the first channel set contains the most amount of vital sign information. By using a softer knowledge space and allowing the projection vector to move within this region, the pulse estimation is more robust to changes in the video itself (e.g., illumination, camera settings, skin color).

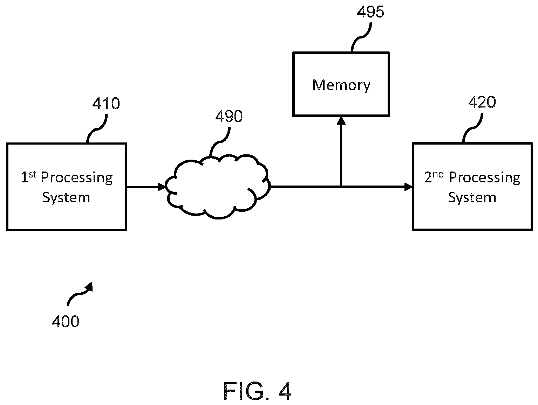

Fig. 4 illustrates a distributed processing system 400 according to an embodiment.

The distributed system comprises a first processing system 410 and a second processing system 420, separate to the first processing system. The first processing system 410 provides a processing system according to an embodiment.

The first processing system 410 is configured to perform any previously described approach for compressing the vital sign image data. The first processing system 410 is configured to transmit the compressed image data to the second processing system 420. This may take place over any suitable communication channel, e.g., over the internet 490.

In some examples, the first processing system 410 is configured to store the compressed image data in a storage system 495 accessible to the second processing system 420. The second processing system 420 may then retrieve the compressed image data to complete the transmittal to the second processing system.

The second processing system is configured to perform a decompression procedure on the compressed image data to produce reconstructed intermediate image data; and process the reconstructed intermediate image data to generate the vital sign information of the subject.

Approaches for performing this process have been previously described.

Embodiments may make use of one or more processing systems.

Any such processing system can be implemented in numerous ways, with software and/or hardware, to perform the various functions required. A processor is one example of a processing system which employs one or more microprocessors that may be programmed using software (e.g., microcode) to perform the required functions. A processing system may however be implemented with or without employing a processor, and also may be implemented as a combination of dedicated hardware to perform some functions and a processor (e.g., one or more programmed microprocessors and associated circuitry) to perform other functions.

Examples of processing system components that may be employed in various embodiments of the present disclosure include, but are not limited to, conventional microprocessors, application specific integrated circuits (ASICs), and field-programmable gate arrays (FPGAs).

In various implementations, a processor or processing system may be associated with one or more storage media such as volatile and non-volatile computer memory such as RAM, PROM, EPROM, and EEPROM. The storage media may be encoded with one or more programs that, when executed on one or more processors and/or processing systems, perform the required functions. Various storage media may be fixed within a processor or processing system or may be transportable, such that the one or more programs stored thereon can be loaded into a processor or processing system.

It will be understood that disclosed methods are preferably computer-implemented methods. As such, there is also proposed the concept of a computer program comprising code means for implementing any described method when said program is run on a processing system, such as a computer. Thus, different portions, lines or blocks of code of a computer program according to an embodiment may be executed by a processing system or computer to perform any herein described method.

In some alternative implementations, the functions noted in the block diagram(s) or flow chart(s) may occur out of the order noted in the figures. For example, two blocks shown in succession may, in fact, be executed substantially concurrently, or the blocks may sometimes be executed in the reverse order, depending upon the functionality involved.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems.

The mere fact that certain measures are recited in mutually different dependent claims does not indicate that a combination of these measures cannot be used to advantage. If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa. Any reference signs in the claims should not be construed as limiting the scope.

**Claims**

1.  A computer-implemented method for compressing vital sign image data, being image data in a first color space from which vital sign information of a subject is derivable, to produce compressed image data for transmittal over a communication channel, the computer-implemented method comprising:

    receiving the vital sign image data;
    transforming the vital sign image data into intermediate image data in a second, different color space, the intermediate image data comprising data in a plurality of channels formed of a first channel set and a second channel set, each channel set comprising one or more channels; and
    performing a compression procedure to produce the compressed image data, the compression procedure comprising:

    performing a first compression process on the data in the first channel set; and
    performing a second compression process on the data in the second channel set,

    wherein the data in the first channel set encodes more vital sign information than the data in the second channel set; and
    wherein the first compression process is less lossy than the second compression process.

2.  The computer-implemented method of claim 1, wherein the first channel set encodes color information of the vital sign image data and the second channel set encodes luminance information of the vital sign image data.

3.  The computer-implemented method of claim 1 or 2, wherein the first compression process is lossless.

4.  The computer-implemented method of any of claims 1 to 3, wherein the step of transforming the vital sign image data comprises processing the vital sign image data using a color space transformation derived from a transformation performed as part of a vital sign extraction procedure performed on vital sign image data.

5.  The computer-implemented method of any of claims 1 to 4, wherein the step of transforming the vital sign image data comprises performing a matrix multiplication on the vital sign image data.

6.  The computer-implemented method of any of claims 1 to 5, wherein the first color space is an RGB color space.

7.  The computer-implemented method of any of claims 1 to 6, further comprising, for each channel of the first channel set,

    identifying values of the intermediate image data, in said channel, that represent exposed skin of the subject;
    determining a maximum value and a minimum value of the identified values; and
    scaling each value in the channel using the maximum value and the minimum value.

8.  The computer-implemented method of claim 7, wherein, for each channel of the first channel set, the step of scaling each value in the channel comprises, for each value:

    subtracting the minimum value from the value to produce a first intermediate value;
    dividing the first intermediate value by the difference between the maximum value and the minimum value to produce a second intermediate value; and
    multiplying the second intermediate value by a predetermined scaling factor.

9.  The computer-implemented method of any of claims 1 to 8, wherein the first compression process is a lossless PNG compression process and the second compression process is a JPEG compression process.

10. A computer-implemented method for generating vital sign information of a subject, the computer-implemented method comprising:

compressing, at a first processing system, vital sign image data by performing the method of any of claims 1 to 9 to produce compressed image data;
transmitting the compressed image data to a second, different processing system;
performing, at the second processing system, a decompression procedure on the compressed image data to produce reconstructed intermediate image data; and
processing the reconstructed intermediate image data to generate vital sign information of the subject.

11. The computer-implemented method of claim 10, wherein the second processing system is a cloud-computing system.

12. The computer-implemented method of claim 10 or 11, wherein processing the reconstructed intermediate image data to generate vital sign information of the subject comprises processing the reconstructed intermediate image data without reconstructing the vital sign image data in the first color space.

13. A computer program product comprising computer program code means which, when executed on a computing device having a processing system, cause the processing system to perform all of the steps of the method according to any of claims 1 to 12.

14. A processing system for compressing vital sign image data, being image data in a second color space from which vital sign information of a subject is derivable, to produce compressed image data,
the processing system being configured to:

receive the vital sign image data;
transform the vital sign image data into intermediate image data in a second, different color space, the intermediate image data comprising data in a plurality of channels formed of a first channel set and a second channel set, each channel set comprising one or more channels; and
perform a compression procedure to produce the compressed image data, the compression procedure comprising:

performing a first compression process on the data in the first channel set; and
performing a second compression process on the data in the second channel set,

wherein, the data in the first channel set is more responsive to changes in the vital sign information than the data in the second channel set; and
wherein the first compression process is less lossy than the second decompression process.

15. A distributed processing system for generating vital sign information of a subject, the distributed processing system comprising a first processing and a second, different processing system,
wherein:

the first processing system is the processing system of claim 14 further configured to transmit the compressed image data to the second processing system;
the second processing system is separate to the first processing system and is configured to perform a decompression procedure on the compressed image data to produce reconstructed intermediate image data; and
process the reconstructed intermediate image data to generate the vital sign information of the subject.

FIG. 1

210

FIG. 2

100

Transmit compressed
image data

310

Decompress

320

Produce vital sign
information

330

300

FIG. 3

FIG. 4

Europäisches
Patentamt

European
Patent Office

Office européen
des brevets

## EUROPEAN SEARCH REPORT

**Application Number**

EP 22 21 5421

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 2 846 550 B1 (KONINKLIJKE PHILIPS NV [NL]) 3 October 2018 (2018-10-03) * paragraphs [0017], [0030] – [0066]; claims; figures * | 1-15 | INV. A61B5/00 A61B5/1455 |
| X | US 2014/206965 A1 (DE HAAN GERARD [NL] ET AL) 24 July 2014 (2014-07-24) * paragraphs [0027] – [0032], [0044] – [0047], [0069] – [0072], [0092] – [0167]; claims; figures * | 1-15 | |
| A | RAPCZYNSKI MICHAL ET AL: "Effects of Video Encoding on Camera-Based Heart Rate Estimation", IEEE TRANSACTIONS ON BIOMEDICAL ENGINEERING, IEEE, USA, vol. 66, no. 12, 1 December 2019 (2019-12-01), pages 3360-3370, XP011757123, ISSN: 0018-9294, DOI: 10.1109/TBME.2019.2904326 [retrieved on 2019-11-19] * left column, first two sentences; page 3364 * | 1-15 | |
| A | WANG WENJIN ET AL: "Algorithmic Principles of Remote PPG", IEEE TRANSACTIONS ON BIOMEDICAL ENGINEERING, IEEE, USA, vol. 64, no. 7, 1 July 2017 (2017-07-01), pages 1479-1491, XP011653906, ISSN: 0018-9294, DOI: 10.1109/TBME.2016.2609282 [retrieved on 2017-06-15] * the whole document * | 1-15 | TECHNICAL FIELDS SEARCHED (IPC) A61B G06T G16H H04N |

The present search report has been drawn up for all claims

| Place of search | Date of completion of the search | Examiner |
|---|---|---|
| The Hague | 26 May 2023 | Crisan, Carmen-Clara |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 21 5421

This annex lists the patent family members relating to the patent documents cited in the above-mentioned European search report.
The members are as contained in the European Patent Office EDP file on
The European Patent Office is in no way liable for these particulars which are merely given for the purpose of information.

26-05-2023

| Patent document cited in search report | | Publication date | Patent family member(s) | | Publication date |
|---|---|---|---|---|---|
| EP 2846550 | B1 | 03-10-2018 | BR 112013017072 | A2 | 14-02-2018 |
| | | | CN 103314583 | A | 18-09-2013 |
| | | | EP 2661885 | A2 | 13-11-2013 |
| | | | EP 2846550 | A1 | 11-03-2015 |
| | | | JP 5940558 | B2 | 29-06-2016 |
| | | | JP 2014506062 | A | 06-03-2014 |
| | | | RU 2013136494 | A | 10-02-2015 |
| | | | US 2013294505 | A1 | 07-11-2013 |
| | | | WO 2012093320 | A2 | 12-07-2012 |
| US 2014206965 | A1 | 24-07-2014 | BR 112014004064 | A2 | 14-03-2017 |
| | | | CN 103765436 | A | 30-04-2014 |
| | | | EP 2748762 | A1 | 02-07-2014 |
| | | | JP 6067706 | B2 | 25-01-2017 |
| | | | JP 2014529439 | A | 13-11-2014 |
| | | | RU 2014111492 | A | 10-10-2015 |
| | | | TR 201910353 | T4 | 22-07-2019 |
| | | | US 2014206965 | A1 | 24-07-2014 |
| | | | US 2017007189 | A1 | 12-01-2017 |
| | | | WO 2013030739 | A1 | 07-03-2013 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **G. DE HAAN ; V. JEANNE.** Robust Pulse Rate from Chrominance-Based rPPG. *IEEE Transactions on Biomedical Engineering,* October 2013, vol. 60 (10), 2878-2886
- **WANG, W. ; DEN BRINKER, A. C. ; STUIJK, S. ; DE HAAN, G.** Algorithmic principles of remote ppg. *IEEE Trans. Biomed. Eng.,* 2016, vol. 64, 1479-1491
- **G. DE HAAN ; A. VAN LEEST.** Improved motion robustness of remote- PPG by using the blood volume pulse signature. *Physiol. Meas.,* October 2014, vol. 35 (9), 1913-1922
- **CHAVES-GONZÁLEZ ; JOSE M. et al.** Detecting skin in face recognition systems: A color spaces study. *Digital signal processing 20.3,* 2010, 806-823
- **ZHANG, SHIFENG et al.** Detecting face with densely connected face proposal network. *Neurocomputing,* 2018, vol. 284, 119-127
- **CHAICHULEE, SITTHICHOK et al.** Cardio-respiratory signal extraction from video camera data for continuous non-contact vital sign monitoring using deep learning. *Physiological measurement 40.11,* 2019, 115001
- **WANG, W. ; DEN BRINKER, A. C. ; STUIJK, S. ; DE HAAN, G.** Algorithmic principles of remote ppg. *IEEE Trans. Biomed. Eng.,* 2016, vol. 64, 1479-1491
- **WANG, W. ; DEN BRINKER, A. C. ; DE HAAN.** Single-Element Remote-PPG. *IEEE Transactions on Biomedical Engineering,* July 2019, vol. 66 (7), 2032-2043